# EUROPEAN PATENT SPECIFICATION

(11) **EP 1 600 122 B1**
(45) Date of publication and mention of the grant of the patent: **01.07.2009**
(21) Application number: 05010494.2
(22) Date of filing: 13.05.2005
(51) Int. Cl.: A61F 2/06, A61L 31/08

(54) **Stent-graft for treatment of aneurysmal tissue**
Stent-Graft zur Behandlung von Aneurysmen
Greffon d'endoprothèse pour le traitement d'aneurysmes

(30) Priority: 27.05.2004 US 855297
(43) Date of publication of application: 30.11.2005
(73) Proprietor: Medtronic Vascular, Inc., Santa Rosa, CA 95403 (US)
(72) Inventor: Fernandes, Brian, Roseville MN 55113 (US); Dinh, Thomas Q., Minnetonka MN 55343 (US); Christoferson, Laura, Ramsey MN 55303 (US); Brin, David S., Topsfield MA 01983-1720 (US); Tseng, David, Princeton Jct. New Jersey 08550 (US)
(74) Representative: Zimmermann & Partner

(56) References cited:
- WO-A-00/56283
- US-A- 5 994 341
- US-A1- 2003 004 564

## Description

### FIELD OF THE INVENTION

The field of the invention is treatment of vascular abnormalities.

### BACKGROUND OF THE INVENTION

Aortic aneurysms pose a significant medical problem for the general population. Aneurysms within the aorta presently affect between two and seven percent of the general population and the rate of incidence appears to be increasing. This form of atherosclerotic vascular disease (hardening of the arteries) is characterized by degeneration in the arterial wall in which the wall weakens and balloons outward by thinning. Until the affected artery is removed or bypassed, a patient with an aortic aneurysm must live with the threat of aortic aneurysm rupture and death.

One clinical approach for patients with an aortic aneurysm is aneurysm repair by endovascular grafting. Endovascular grafting involves the transluminal placement of a prosthetic arterial stent graft in the endoluminal position (within the lumen of the artery). To prevent rupture of the aneurysm, a stent graft of tubular construction is introduced into the aneurysmal blood vessel, typically from a remote location through a catheter introduced into a major blood vessel in the leg.

When inserted and deployed in a vessel, a stent graft keeps the vessel open. The stent graft typically has the form of an open-ended tubular element and most frequently is configured to enable its expansion from an outside diameter which is sufficiently small to allow the stent graft to traverse the vessel to reach a site where it is to be deployed, to an outside diameter sufficiently large to engage the inner lining of the vessel for retention at the site.

Despite the effectiveness of endovascular grafting, once the aneurysmal site is bypassed, the aneurysm remains. The aortic tissue can continue to degenerate such that the aneurysm increases in size due to thinning of the medial connective tissue architecture of the aorta and loss of elastin. Two processes, namely over-expression of matrix metalloproteinases (MMPs) and inflammation, are commonly cited as major players in the propagation of aneurysmal formation. In the diseased aortic tissue, the tightly-controlled balance between MMPs and tissue inhibitors of MMPs is severely disrupted and the result is increased local and plasma levels of several MMPs. Of these, it has been determined that MMP-2 and MMP-9 are primarily responsible for the elastin and collagen degradation that precedes aortic expansion and dilatation.

Chronic inflammation also is a hallmark of aneurysmal degeneration. The inflammatory response in aneurysm pathology is essentially transmural in distribution, with dense infiltrates found in the media and adventitial portion of the vessel. While the specific factors that initiate the process are uncertain, the recruitment of inflammatory cells can essentially be ascribed to various inflammatory mediators. In addition, chronic inflammation is often accompanied by an angiogenic response.

There is a desire in the art to achieve a greater success of aneurysm repair and healing. It is therefore an object of the present invention to overcome the problems associated with the prior art at least partially.

Vascular Implants which are coated with a variety of therapeutic agents are known for example from US-A-5 994 341.

### SUMMARY OF THE INVENTION

This object is solved by a treatment device according to claim 1. Further aspects, features, advantages and details of the present invention are apparent from the dependent claims, the description, and the accompanying drawings. Embodiments according to the present invention address the problem of aneurysm repair, particularly the problem of continued breakdown of aortic aneurysmal tissue even after deployment of a stent graft. A consequence of such continued breakdown is rupture of the aneurysm. Methods and apparatus for supporting or bolstering the aneurysmal site by implanting a stent graft, while supplying a pharmaceutical agent to aid in stabilizing and healing the aneurysmal tissue, are provided.

Thus, in one embodiment according to the invention there is provided an intravascular treatment device, comprising a stent graft locatable adjacent to an aneurysmal site where the stent includes a time-release coating consisting essentially of a polymer and two or more therapeutic agents. In some embodiments of the invention, the polymer of the coating is biodegradable. In other embodiments, the coating of the polymer is not biodegradable. In other embodiments, the polymer is a pH- or temperature-sensitive polymer. Therapeutic agents that can be used include two therapeutics, selected from a matrix metalloproteinase inhibitor, cyclooxygenase-2 inhibitor, anti-adhesion molecule, tetracycline-related compound, beta blocker, NSAID, steroidal anti-inflammatory or angiotensin converting enzyme inhibitor. According to the invention, the coating comprises both an anti-inflammatory and a matrix metalloproteinase inhibitor.

### BRIEF DESCRIPTION OF THE DRAWINGS

Understanding the invention may be had by reference to the embodiments according to the invention described in the present specification and illustrated in the appended drawings.

Figure 1 is a schematic view of a human aortal aneurysm.

Figure 2 is a partial sectional view of a descending aorta with a bifurcated stent graft placed therein.

Figure 3, is a graph showing the effect of different stimulants on MMP-9 release from human-derived white blood cells.

Figure 4A is a graph showing the effect of doxycyline on MMP-9 secretion from PHA-stimulated human white blood cells using one experimental procedure. Figure 4B is a graph showing the effect of doxycycline on MMP-9 secretion from PHA-stimulated human white blood cells using an alternative experimental procedure.

Figure 5A is a graph showing the results of an MMP-9 assay. Figure 5B is a graph showing the results of an IL-1α assay.

### DETAILED DESCRIPTION

Reference will now be made in detail to exemplary embodiments according to the invention.

Methods and apparatus for stabilizing and treating an aneurysmal site include implanting a coated endovascular stent graft that delivers a bioactive amount of one or more therapeutic agents to the aneurysmal site, preferably at least two therapeutic agents, and according to the invention including both an anti-inflammatory and a metalloproteinase. The stent graft is implanted in an individual in a typical manner, where the stent graft acts as a delivery vehicle to deliver the one or more therapeutic agents to the aneurysmal site. Specifically, a compound comprised of a suitable polymer and the one or more therapeutic agent or agents is used to coat the stent graft.

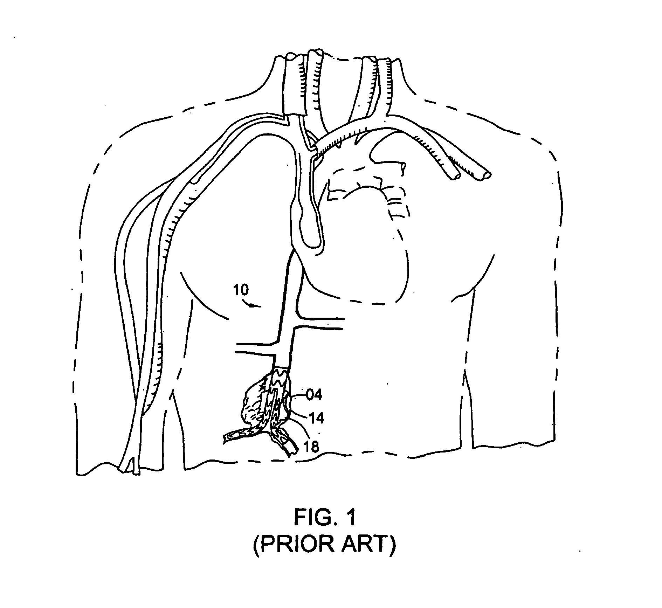

Referring initially to Figure 1, there is shown generally an aneurysmal blood vessel; in particular, there is an aneurysm of the aorta 10, such that the aorta or blood vessel wall 04 is enlarged at an aneurysmal site 14 and the diameter of the aorta 10 at the aneurysmal site 14 is on the order of over 150% to 300% of the diameter of a healthy aorta. The aneurysmal site 14 forms an aneurysmal bulge or sac 18. If left untreated, the aneurysmal sac 18 may continue to deteriorate, weaken, increase in size, and eventually tear or burst.

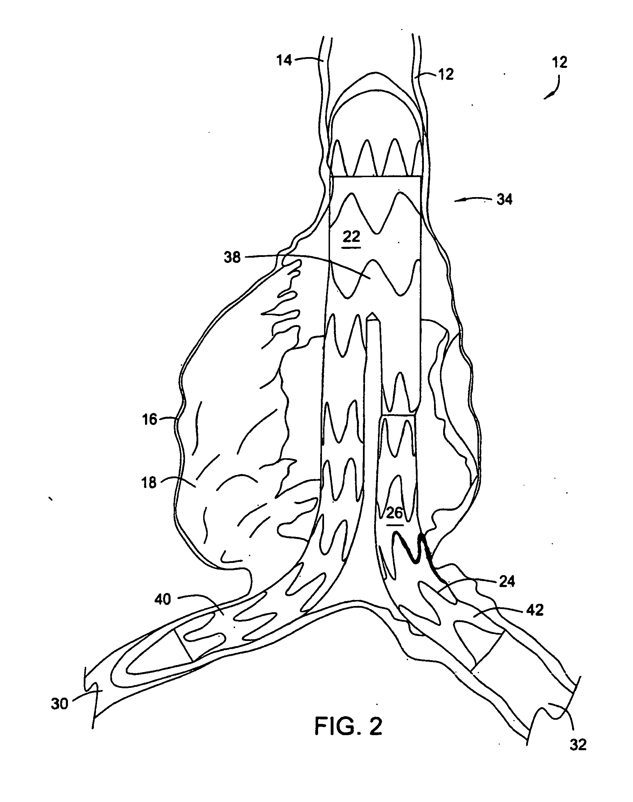

Figure 2 shows a stent graft 22 deployed within an aorta 12. The stent graft 22 typically includes a stent portion 24, having a structurally supportive yet collapsible construction, to which a graft portion 26 is sewn or attached. The stent portion 24 provides a tubular body having a support capability sufficient to hold the graft portion 26 in an open position across the aneurysmal sac 18, such that the opposed ends are received and sealed against healthy portions 14 of the of the aorta. The graft portion 26 blocks the passage of blood to the aneurysmal sac 18, and provides a conduit for blood flow past the aneurysmal sac 18.

Although the stent graft 22 provides an exclusionary environment through which blood may flow past the aneurysmal sac 18, there remains a need to treat the aneurysmal sac 18. In particular, it is known that fresh blood may leak into the aneurysmal sac 18 region despite the presence of stent graft 22, leading to further breakdown in the extracellular matrix of the aneurysmal vessel. If this occurs, the excluded aneurysmal vessel may rupture leading to patient mortality. Therefore, there remains a need to treat the aneurysmal sac 18 further despite the presence of the excluding device or as an alternative to using an excluding device.

One or more therapeutic agents described herein, *infra,* are provided with an excluding device or intravascular repair vehicle, for example, the stent graft 22 shown in Figure 2. Referring back to Figure 2, the placement of the stent graft 22 in the aorta 10 is a technique well known to those skilled in the art, and essentially includes the opening of a blood vessel in the leg, and the insertion of the stent graft 22 contained in a catheter into the vessel, guiding the catheter through the vessel, and deploying the stent graft 22 in a position spanning the aneurysmal sac 18.

Although in Figure 2 the bifurcated stent graft 22 is shown in its fully assembled and positioned state, it is to be understood that the bifurcated stent graft 22 typically has at least three portions, a trunk portion 38, located in the lower portion of the ascending aorta, and two minor diameter leg portions 40, 42 that fit into the two iliac arteries 30 and 32. In one embodiment, the bifurcated stent graft 22 is configured such that each trunk and leg portion 38, 40 and 42 includes a graft portion, supported externally by a tubular metal stent portion that expands to a pre-established diameter when placed in the aorta.

When assembled *in situ,* the entire stent graft 22 spans the aneurysmal sac 18 in the aorta 10, to seal the aneurysmal portion of the aorta 10 from blood flowing through the aorta 10. The metal stent 24 includes a plurality of hoop frame members each of which preferably includes a plurality of elements, here, diamond-shaped. The procedure and attachment mechanisms for assembling the stent graft 10 in place in this configuration are well known in the art, and are disclosed in, e.g., Lombardi, et al., US Pat No. 6,203,568.

The coating compound used in embodiments according to the invention is adapted to exhibit a combination of physical characteristics such as biocompatibility, and, preferably, biodegradability and bio-absorbability, provided on a delivery vehicle such as stent graft. The coating compound allows for release of the therapeutic agents that aid in the treatment of aneurysmal tissue. The coating compound used is biocompatible such that it results in no induction of inflammation or irritation when implanted, degraded or absorbed.

The therapeutic agent/coating formulation comprises a material to ensure the controlled release of the therapeutic agent. The materials to be used for such a coating preferably are comprised of a biocompatible polymer in which the therapeutic agent or agents are present. The location of the therapeutic agent on the delivery vehicle allows the therapeutic reaction to be substantially localized so that overall dosages to the individual can be reduced and undesirable side effects caused by the action of the agent or agents in other parts of the body are minimized.

Thus, the therapeutic agent formulation comprises a carrier, which according to the present invention can be made of synthetic polymers, natural polymers, inorganics and combinations of these. The polymers may be either biodegradable or non-biodegradable. Typical examples of biodegradable synthetic polymers are listed below:
- Aliphatic polyesters, such as poly(lactic acid), poly(glycolic acid), poly(lactic acid-co-glycolic acid), poly(ε-caprolactone), poly(trimethylene carbonate), polydioxanone and copolymers; poly(hydroxy butyrate) (Biopol®), poly(hydroxy valerate), poly(hydroxy butyrate-co-hydroxy valerate), poly(butylene succinate) (Bionolle®), poly(butylene adipate),
- Polyanhydrides, such as poly(adipic anhydride), and poly(sebacic acid-co-1,3-bis(p-carboxyphenoxy)propane),
- Poly(ortho ester)s,
- Poly(ester amide)s, such as based on 1,4-butanediol, adipic acid, and 1,6-aminohexanoic acid (BAK 1095),
- Poly(ester urethane)s,
- Poly(ester anhydride)s,
- Poly(ester carbonate)s, such as tyrosine-poly(alkylene oxide)-derived poly(ether carbonate)s,
- Polyphosphazenes,
- Polyarylates, such as tyrosine-derived polyarylates,
- Poly(ether ester)s, such as poly(butylene terephthalate)-poly(ethylene glycol) copolymers (PolyActive®), poly(ε-caprolactone)-b-poly(ethylene glycol)) block copolymers, and poly(ethylene oxide)-b-poly(hydroxy butyrate) block copolymers.

Examples of biostable synthetic polymers include:
- Polyolefins, such as polyethylene, polypropylene,
- Polyurethanes,
- Fluorinated polyolefins, such as polytetrafluorethylene (Teflon®),
- Chlorinated polyolefins, such as poly(vinyl chloride),
- Polyamides,
- Acrylate polymers, such as poly(methyl methacrylate) and copolymers (Eudragit®),
- Acrylamide polymers, such as poly(N-isopropylacrylamide),
- Vinyl polymers, such as poly(N-vinylpyrrolidone), poly(vinyl alcohol), poly(vinyl acetate), poly(ethylene-co-vinylacetate),
- Polyacetals,
- Polycarbonates,
- Polyethers, such as based on poly(oxyethylene) and poly(oxypropylene) units (Pluronic®),
- Aromatic polyesters, such as poly(ethylene terephthalate) (Dacron®), poly(propylene terephthalate) (Sorona®)
- Poly(ether ether ketone)s,
- Polysulfones,
- Silicone rubbers,
- Thermosets, such as epoxies,
- and Poly(ester imide)s

Representative examples of inorganics are listed below:
- Hydroxyapatite,
- Tricalcium phosphate,
- Silicates, such as Bioglass®, montmorillonite, and mica.

Typical examples of natural polymers include albumin, collagen, gelatin, hyaluronic acid, elastin, chondroitin sulfate, chitin, chitosan, curdlan, carrageenan, starch, alginate, alternan, elsinan, emulsan, gellan, glycogen, glycolipids, glycopeptides, pectin, pullularn, inulin, succinoglycan, xanthan, cellulose and cellulose derivatives (such as methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextran, polysaccharides (such as sucrose acetate isobutyrate), and fibrin.

In general, see United States Pat. Nos. 6,514,515 to Williams; 6,506,410 to Park, et al.; 6,531,154 to Mathiowitz, et al.; 6,344,035 to Chudzik, et al.; 6,376,742 to Zdrahala, et al.; and Griffith, L.A., Ann. N.Y. Acad. of Sciences, 961:83-95 (2002); and Chaikof, et al, Ann. N.Y. Acad. of Sciences, 961:96-105 (2002). The polymers as described herein can also be blended or copolymerized in various compositions as required.

The polymeric coatings as discussed can be fashioned in a variety of forms with desired release characteristics and/or with specific desired properties. For example, the polymeric coatings may be fashioned to release the therapeutic agent or agents upon exposure to a specific triggering event such as pH. Representative examples of pH-sensitive polymers include poly(acrylic acid) and its derivatives (including for example, homopolymers such as poly(aminocarboxylic acid); poly(acrylic acid); poly(methyl acrylic acid), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropyl methylcellulose acetate succinate; cellulose acetate trimellilate; and chitosan. Yet other pH sensitive polymers include any mixture of a pH sensitive polymer and a water-soluble polymer.

Likewise, polymeric carriers can be fashioned that are temperature sensitive. Representative examples of thermogelling polymers and their gelatin temperature include homopolymers such as poly(N-methyl-N-n-propylacrylamide)(19.8°C); poly(N-n-propylacrylamide)(21.5°C); poly(N-methyl-N-isopropylacrylamide)(22.3°C); poly(N-n-propylmethacrylamide(28.0°C); poly(N-isopropylacrylamide)(30.9°C); poly(N,n-diethylacrylamide)(32.0°C); poly(N-isopropylmethacrylamide)(44.0°C); poly(N-cyclopropylacrylamide)(45.5°C); poly(N-ethylmethyacrylamide)(50.0°C); poly(N-methyl-N-ethylacrylamide)(56.0°C); poly(N-cyclopropylmethacrylamide)(59.0°C); poly(N-ethylacrylamide)(72.0°C). Moreover, thermogelling polymers may be made by preparing copolymers between (among) monomers of the above, or by combining such homopolymers with other water-soluble polymers such as acrylmonomers (e.g., acrylic acid and derivatives thereof such as methylacrylic acid, acrylate and derivatives thereof such as butyl methacrylate, acrylamide, and N-n-butyl acrylamide).

Other representative examples of thermogelling polymers include cellulose ether derivatives such as hydroxypropyl cellulose (41°C); methyl cellulose (55°C); hydroxypropylmethyl cellulose (66°C); and ethylhydroxyethyl cellulose, and Pluronics such as F-127 (10-15 °C); L-122 (19°C); L-92 (26°C); L-81 (20°C); and L-61 (24°C).

For information regarding stents and coatings, see U.S. Pat. Nos. 6,387,121 to Alt; 6,451,373 to Hossainy, et al.; and 6,364,903 to Tseng, et al. which are exemplary of coatings on stent grafts.

Generally, aneurysms result from the invasion of the cell wall by elastin-attacking proteins that occur naturally in the body, but for unknown reasons begin to congregate at certain blood vessel sites, attack the blood vessel structure and cause inflammation of the vessel. Generally, a plurality of enzymes, proteins and acids--all naturally occurring--interact through specific biochemical pathways to form elastin-attacking proteins or to promote the attachment or absorption of elastin-attacking proteins into the cell wall. The elastin-attacking proteins and the resulting breakdown of tissue and inflammation are leading causes of aneurysm formation.

For example, arachidonal acid, a naturally-occurring fatty acid found in blood vessel, forms the cytokine PGE2 in the presence of COX-2. PGE2, when taken up by macrophages, induces the expression of matrix metalloproteinase 9 (MMP-9), which is an etastin-attacking enzyme and a member of a family of metalloproteinases. Additionally, PGE2 is believed to contribute directly to inflammation in the blood vessel wall, furthering physiological stress in the blood vessel wall. Moreover, each of these elastin-attacking protein compounds is likely to be present on the surface of the blood vessel wall, as well as within the cellular matrix of the blood vessel. Thus, agents that work to reduce the attachment and integration of elastin-attacking and inflammatory compounds to the blood vessel also are of interest for use as a therapeutic.

The therapeutic agents described provide intervention in the aforementioned biochemical pathways and mechanisms, reduction in the level of the individual components responsible for aneurysmal growth, and elimination or limitation of the advance of the aneurysmal event. In particular, therapeutic agents are provided, alone or in combination, to address the inflammation- or elastin-attacking compounds, which cause the transition of a blood vessel from a healthy to an aneurysmal condition. The therapeutic agent or agents are released over time in the aneurysmal location, reducing the likelihood of further dilation and increasing the likelihood of successful repair of the aneurysm.

The therapeutic agents described are those useful in suppressing proteins known to occur in and contribute to aneurysmal sites, reducing inflammation at the aneurysmal site, and reducing the adherence of elastin-attacking proteins at the aneurysmal site. For example one class of materials, matrix metallproteinase (MMP) inhibitors, have been shown in some cases to reduce such elastin-attacking proteins directly, or in other cases indirectly by interfering with a precursor compound needed to synthesize the elastin-attacking protein. Another class of materials, non-steroidal anti-inflammatory drugs (NSAlDs), has demonstrated anti-inflammatory qualities that reduce inflammation at the aneurysmal site, as well as an ability to block MMP-9 formation. Steroidal anti-inflammatory drugs such as dexamethasone, beclomethasone and the like, may also be used to reduce inflammation. Further, yet another class of agents, attachment inhibitors, prevent or reduce the attachment or adherence of elastin-attacking proteins or inflammation-causing compounds onto the vessel wall at the aneurysmal site. Thus, these therapeutic agents and other such agents, alone or in combination, when provided at an aneurysmal site directly affect or undermine the underlying sequence of events leading to aneurysm formation and progression.

One class of agents useful in this application are those that block the formation of MMP-9 by interfering with naturally occurring body processes which yield MMP-9 as a byproduct. Cyclooxygenase-2 or "COX-2" is known to metabolize a fat in the body known as arachidonic acid or AA, a naturally occurring omega-6 fatty acid found in nearly all cell membranes in humans. Prostaglandin E2 (PGE2) is synthesized from the catalyzation of COX-2 and arachidonic acid and, when PGE2 is taken up by macrophages, it results in MMP-9 formation. Thus, if any of COX-2, PGE2, or AA is suppressed, then MMP-9 formation will be suppressed. Therefore, COX-2 inhibitors can be provided at the aneurysmal site. Such COX-2 inhibitors include Celecoxib, Rofecoxib and Parecoxib, all of which are available in pharmacological preparations. Additionally, COX-2 inhibition has been demonstrated from administration of herbs such as green tea, ginger, turmeric, chamomile, Chinese gold-thread, barberry, baikal skullcap, Japanese knotweed, rosemary, hops, feverfew, and oregano; and other agents such as piroxican, mefenamic acid, meloxican, nimesulide, diclofenac, MF-tricyclide, raldecoxide, nambumetone, naproxen, herbimycin-A, and etoicoxib, and it is specifically contemplated as an embodiment according to the present invention that such additional COX-2 inhibiting materials may be formulated for use in an aneurysmal location.

In addition to inhibiting COX-2 formation, the generation of elastin-attacking proteins may be limited by interfering with the oxidation reaction between COX-2 and AA by reducing the capability of AA to oxidize. It is known that certain NSAIDs provide this function. For example, ketoralac tromethamine (Toradol) inhibits synthesis of progstaglandins including PGE2. In addition, other currently available NSAIDs, including indomethacin, ketorolac, ibuprofen and aspirin, among others, reduce inflammation at the aneurysmal site, limiting the ability of elastin attacking proteins such as MMP-9 to enter into the cellular matrix of the blood vessel and degrade elastin. Additionally, steroidal based anti-inflammatories, such as methylprednisolone or dexamethasone may be provided to reduce the inflammation at the aneurysmal site.

Despite the presence of inhibitors of COX-2 or of the oxidation reaction between COX-2 and AA; and/or despite the presence of an anti-inflammatory to reduce irritation and swelling of the blood vessel wall, MMP-9 may still be present in the blood vessel. Therefore, another class of therapeutic agents useful in this application is the class that limits the ability of elastin-attacking proteins to adhere to the blood vessel wall, such as anti-adhesion molecules. Anti-adhesion molecules, such as anti-CD18 monoclonal antibody, limit the capability of leukocytes that may have taken up MMP-9 to attach to the blood vessel wall, thereby preventing MMP-9 from having the opportunity to enter into the blood vessel cellular matrix and attack the elastin.

In addition, other therapeutic agents contemplated to be used to inhibit MMP-9 are tetracycline and related tetracycline-derivative compounds. In using a tetracycline compound as a bioactive agent in aneurysm treatment, the observed anti-aneurysmal effect appears to be unrelated to and independent of any antimicrobial activity such a compound might have. Accordingly, the tetracycline may be an antimicrobial tetracycline compound, or it may be a tetracycline analogue having little or no significant antimicrobial activity.

Preferred antimicrobial tetracycline compounds include, for example, tetracycline per se, as well as derivatives thereof. Preferred derivatives include, for example, doxycycline (4-(Dimethylamino)-1, 4, 4a, 5, 5a, 6, 11, 12a-octahydro-3,5, 10, 12, 12a-pentahydroxy-6-methyl-1, 11-dioxo-2-naphthacenecarboxamide monohydrate), aureomycin and chloromycin. If a tetracycline analogue having little or no antimicrobial activity is to be employed, it is preferred that the compound lack the dimethylamino group at position 4 of the ring structure. Such chemically-modified tetracyclines include, for example, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, and 6α-deoxy-5-hydroxy-4-dedimethylaminotetracycline. Also, tetracyclines modified at the 2-carbon position to produce a nitrile, e.g., tetracyclinonitrile, are useful as non-antibacterial, anti-metalloproteinase agents. Further examples of tetracyclines modified for reduced antimicrobial activity include 6-α-benzylthiomethylenetetracycline, the mono-N-alkylated amide of tetracycline, 6-fluoro-6-demethyltetracycline, and 11-α-chlorotetracycline.

Among the advantages of embodiments according to the present invention is that the tetracycline compound is administered locally in an amount which has substantially no antibacterial activity, but which is effective for reducing pathology for inhibiting the undesirable consequences associated with aneurysms in blood vessels. Alternatively, as noted above, the tetracycline compound can have been modified chemically to reduce or eliminate its antimicrobial properties. The use of such modified tetracyclines is preferred in embodiments according to the present invention since they can be used at higher levels than antimicrobial tetracyclines, while avoiding certain disadvantages, such as the indiscriminate killing of beneficial microbes that often accompanies the use of antimicrobial or antibacterial amounts of such compounds.

Another class of therapeutic agent that finds utility in inhibiting the progression of or inducing the regression of a pre-existing aneurysm is beta blockers or beta adrenergic blocking agents. Beta blockers are bioactive agents that reduce the symptoms associated with hypertension, cardiac arrhythmias, angina pectoris, migraine headaches, and other disorders related to the sympathetic nervous system. Beta blockers also are often administered after heart attacks to stabilize the heartbeat. Within the sympathetic nervous system, beta-adrenergic receptors are located mainly in the heart, lungs, kidneys and blood vessels. Beta-blockers compete with the nerve-stimulated hormone epinephrine for these receptor sites and thus interfere with the action of epinephrine, lowering blood pressure and heart rate, stopping arrhythmias, and preventing migraine headaches. Because it is also epinephrine that prepares the body for "fight or flight", in stressful or fearful situations, beta-blockers are sometimes used as anti-anxiety drugs, especially for stage fright and the like. There are two main beta receptors, beta 1 and beta 2. Some beta blockers are selective, such that they selectively block beta 1 receptors. Beta 1 receptors are responsible for the heart rate and strength of the heartbeat. Nonselective beta blockers block both beta 1 and beta 2 receptors. Beta 2 receptors are responsible for the function of smooth muscle.

Beta blockers that may be used in the compounds and methods according to the present invention include acebutolol, atenolol, betaxolol, bisoprolol, carteolol, carvedilol, esmolol, labetolol, metoprolol, nadolol, penbutolol, pindolol, propranolol, and timolol, as well as other beta blockers known in the art.

In addition to therapeutic agents that inhibit elastases or reduce inflammation are agents that inhibit formation of angiotensin II, known as angiotensin converting enzyme (ACE) inhibitors. ACE inhibitors are known to alter vascular wall remodeling, and are used widely in the treatment of hypertension, congestive heart failure, and other cardiovascular disorders. In addition to ACE inhibitors' antihypertensive effects, these compounds are recognized as having influence on connective tissue remodeling after myocardial infarction or vascular wall injury.

ACE inhibitors prevent the generation of angiotensin-II, and many of the effects of angiotensin-II involve activation of cellular AT1 receptors; thus, specific AT1 receptor antagonists have also been developed for clinical application. ACE is an ectoenzyme and a glycoprotein with an apparent molecular weight of 170,000 Da. Human ACE contains 1277 amino acid residues and has two homologous domains, each with a catalytic site and a region for binding Zn⁺². ACE has a large amino-terminal extracellular domain and a 17-amino acid hydrophobic stretch that anchors the ectoenzyme to the cell membrane. Circulating ACE represents membrane ACE that has undergone proteolysis at the cell surface by a sectretase.

ACE is a rather nonspecific enzyme and cleaves dipeptide units from substrates with diverse amino acid sequences. Preferred substrates have only one free carboxyl group in the carboxyl-terminal amino acid, and proline must not be the penultimate amino acid. ACE is identical to kininase II, which inactivates bradkinin and other potent vasodilator peptides. Although slow conversion of angiotensin I to angiontensin II occurs in plasma, the very rapid metabolism that occurs in vivo is due largely to the activity of membrane-bound ACE present on the luminal aspect of the vascular system--thus, the localized delivery of the ACE inhibitor contemplated in embodiments according to the present invention provide a distinct advantage over prior art systemic modes of administration.

Following the understanding of ACE, research focused on ACE inhibiting substances to treat hypertension. The essential effect of ACE inhibitors is to inhibit the conversion of relatively inactive angiotensin I to the active angiotensin II. Thus, ACE inhibitors attenuate or abolish responses to angiotensin I but not to angiotensin II. In this regard, ACE inhibitors are highly selective drugs. They do not interact directly with other components of the angiotensin system, and the principal pharmacological and clinical effects of ACE inhibitors seem to arise from suppression of synthesis of angiotensin II. Nevertheless, ACE is an enzyme with many substrates, and systemic administration of ACE inhibitors may not be optimal.

Many ACE inhibitors have been synthesized. Many ACE inhibitors are ester-containing prodrugs that are 100 to 1000 times less potent ACE inhibitors than the active metabolites but have an increased bioavailability for oral administration than the active molecules.

Currently, twelve ACE inhibitors are approved for used in the United States. In general, ACE inhibitors differ with regard to three properties: (1) potency; (2) whether ACE inhibition is due primarily to the drug itself or to conversion of a prodrug to an active metabolite; and (3) pharmacokinetics (i.e., the extent of absorption, effect of food on absorption, plasma half-life, tissue distribution, and mechanisms of elimination). For example, with the notable exceptions of fosinopril and spirapril, which display balanced elimination by the liver and kidneys, ACE inhibitors are cleared predominantly by the kidneys. Therefore, impaired renal function inhibits significantly the plasma clearance of most ACE inhibitors, and dosages of such ACE inhibitors should be reduced in patients with renal impairment.

For systemic administration there is no compelling reason to favor one ACE inhibitor over another, since all ACE inhibitors effectively block the conversion of angiotensin I to angiontensin II and all have similar therapeutic indications, adverse-effect profiles and contraindications. However, there are preferred ACE inhibitors for use in embodiments according to the present invention. ACE inhibitors differ markedly in their activity and whether they are administered as a prodrug, and this difference leads to identify the preferred locally-delivered ACE inhibitors.

One preferred ACE inhibitor is captopril (Capoten). Captopril was the first ACE inhibitor to be marketed, and is a potent ACE inhibitor with a Ki of 1.7 nM. Captopril is the only ACE inhibitor approved for use in the United States that contains a sulfhydryl moiety. Given orally, captopril is rapidly absorbed and has a bioavailability of about 75%. Peak concentrations in plasma occur within an hour, and the drug is cleared rapidly with a half-life of approximately 2 hours. The oral dose of captopril ranges from 6.25 to 150 mg two to three times daily, with 6.25 mg three times daily and 25 mg twice daily being appropriate for the initiation of therapy for heart failure and hypertension, respectively.

Another preferred ACE inhibitor is lisinopril. Lisinopril (Prinivil, Zestril) is a lysine analog of enalaprilat (the active form of enalapril (described below)). Unlike enalapril, lisinopril itself is active. In vitro, lisinopril is a slightly more potent ACE inhibitor than is enalaprilat, and is slowly, variably, and incompletely (about 30%) absorbed after oral administration; peak concentrations in the plasma are achieved in about 7 hours. Lisinopril is cleared as the intact compound in the kidney, and its half-life in the plasma is about 12 hours. Lisinopril does not accumulate in the tissues. The oral dosage of lisinopril ranges from 5 to 40 mg daily (single or divided dosage), with 5 and 10 mg daily being appropriate for the initiation of therapy for heart failure and hypertension, respectively.

Enalapril (Vasotec) was the second ACE inhibitor approved in the United States. However, because enalapril is a prodrug that is not highly active and must be hydrolyzed by esterases in the liver to produce enalaprilat, the active form, enalapril is not a preferred ACE inhibitor of the present invention. Similarly, fosinopril (Monopril), benazepril (Lotensin), fosinopril (Monopril), trandolapril (Mavik) (quinapril (Accupril), ramipril (Altace), moexipirl (Univasc) and perindopril (Aceon) are all prodrugs that require cleavage by hepatic esterases to transform them into active, ACE-inhibiting forms, and are not preferred ACE inhibitors. However, the active forms of these compounds (i.e., the compounds that result from the prodrugs being converted by hepatic esterases)--namely, enalaprilat (Vasotec injection), fosinoprilat, benazeprilat, trandolaprilat, quinaprilat, ramiprilat, moexiprilat, and perindoprilat―are suitable for use, and because of the localized drug delivery, the bioavailability issues that affect the oral administration of the active forms of these agents are moot.

The maximal dosage of the therapeutic or combination of therapeutics to be administered is the highest dosage that effectively inhibits elastolytic, inflammatory or other aneurysmal activity, but does not cause undesirable or intolerable side effects. For example, a tetracycline compound can be administered in an amount of from about 0.1 mg/kg/day to about 30 mg/kg/day, and preferably from about 1 mg/kg/day to about 18 mg/kg/day. For the purpose of embodiments according to the present invention, side effects include clinically significant antimicrobial or antibacterial activity, as well as toxic effects. For example, a dose in excess of about 50 mg/kg/day would likely produce side effects in most mammals, including humans. The dosage of the therapeutic agent or agents used will vary depending on properties of the coating, including its time-release properties, whether the coating is itself biodegradable, and other properties. Also, the dosage of the therapeutic agent or agents used will vary depending on the potency, pathways of metabolism, extent of absorption, half-life, and mechanisms of elimination of the therapeutic agent itself. In any event, the practitioner is guided by skill and knowledge in the field, and embodiments according to the present invention include without limitation dosages that are effective to achieve the described phenomena.

The therapeutic agent or agents may be linked by occlusion in the matrices of the polymer coating, bound by covalent linkages, or encapsulated in microcapsules. Within certain embodiments, the therapeutic agent or agents are provided in noncapsular formulations such as microspheres (ranging from nanometers to micrometers in size), pastes, threads of various size, films and sprays.

Within certain aspects, the coating is formulated to deliver the therapeutic agent or agents over a period of several hours, days, or, months. For example, "quick release" or "burst" coatings are provided that release greater than 10%, 20%, or 25% (w/v) of the therapeutic agent or agents over a period of 7 to 10 days. Within other embodiments, "slow release" therapeutic agent or agents are provided that release less than 1% (w/v) of a therapeutic agent over a period of 7 to 10 days. Further, the therapeutic agent or agents of the present invention should preferably be stable for several months and capable of being produced and maintained under sterile conditions.

Within certain aspects, therapeutic coatings may be fashioned in any size ranging from 50 nm to 500 µm, depending upon the particular use. Alternatively, such compositions may also be readily applied as a "spray", which solidifies into a film or coating. Such sprays may be prepared from microspheres of a wide array of sizes, including for example, from 0.1 µm to 3 µm, from 10 µm to 30 µm, and from 30 µm to 100 µm.

The therapeutic agent or agents according to the present invention also may be prepared in a variety of "paste" or gel forms. For example, within one embodiment of the invention, therapeutic coatings are provided which are liquid at one temperature (e.g., temperature greater than 37°C, such as 40°C, 45°C, 50°C, 55°C or 60°C), and solid or semi-solid at another temperature (e.g., ambient body temperature, or any temperature lower than 37°C). Such "thermopastes" readily may be made utilizing a variety of techniques. Other pastes may be applied as a liquid, which solidify in vivo due to dissolution of a water-soluble component of the paste and precipitation of encapsulated drug into the aqueous body environment.

Within yet other aspects, the therapeutic compositions according to the present invention may be formed as a film. Preferably, such films are generally less than 5, 4, 3, 2, or 1 mm thick, more preferably less than 0.75 mm, 0.5 mm, 0.25 mm, or, 0.10 mm thick. Films can also be generated of thicknesses less than 50 µm, 25 µm or 10 µm. Such films are preferably flexible with a good tensile strength (e.g., greater than 50, preferably greater than 100, and more preferably greater than 150 or 200 N/cm²), have good adhesive properties (i.e., adhere to moist or wet surfaces), and have controlled permeability.

Within certain embodiments, the therapeutic compositions may also comprise additional ingredients such as surfactants (e.g., pluronics, such as F-127, L-122, L-101, L-92, L-81, and L-61).

In one embodiment, the coating is coated with a physical barrier. Such barriers can include inert biodegradable materials such as gelatin, PLGA/MePEG film, PLA, or polyethylene glycol among others. In the case of PLGA/MePEG, once the PLGA/ MePEG becomes exposed to blood, the MePEG will dissolve out of the PLGA, leaving channels through the PLGA to underlying layer of biologically active substance (e.g., poly-1-lysine, fibronectin, or chitosan), which then can initiate its biological activity.

In one embodiment, the coating mix is comprised of multiple layers of coatings with each layer containing one or more therapeutic agents.

Protection of the therapeutic coating also can be utilized by coating the surface with an inert molecule that prevents access to the active site through steric hindrance, or by coating the surface with an inactive form of the biologically active substance, which is later activated. For example, the coating further can be coated readily with an enzyme, which causes either release of the therapeutic agent or agents or activates the therapeutic agent or agents. Indeed, alternating layers of the therapeutic coating with a protective coating may enhance the time-release properties of the coating overall.

Another example of a suitable second coating is heparin, which can be coated on top of therapeutic agent-containing coating. The presence of heparin delays coagulation. As the heparin or other anticoagulant dissolves away, the anticoagulant activity would stop, and the newly exposed therapeutic agent-containing coating could initiate its intended action.

In another strategy, the stent graft can be coated with an inactive form of the therapeutic agent or agents, which is then activated once the stent graft is deployed. Such activation could be achieved by injecting another material into the aneurysmal sac after the stent graft is deployed. In this iteration, the graft material could be coated with an inactive form of the therapeutic agent or agents, applied in the usual manner. Prior to the deployment of the aortic segment of the device, a catheter would be placed within the aneurysm sac via the opposite iliac artery, via an upper limb vessel such as a brachial artery, or via the same vessel as the aortic segment is inserted through so that once the stent graft is deployed, this catheter will be inside the aneurysm sac, but outside the stent graft. The stent graft would then be deployed in the usual manner. Once the stent graft is fully deployed, excluding the aneurysm, the activating substance is injected into the aneurysm sac around the outside of the stent graft.

### EXAMPLES

### I. Cytokine Assay Procedures

*In vitro* assessment of the profile of cytokine expression is a valuable initial screening tool to assess the likely outcome of inflammatory reactions *in vivo.* The protocol described herein outlines a method to assess the inflammatory response of activated human blood-derived cells to potential drug candidates. A short protocol can be adopted if a total white blood cell population is desired. Alternatively, a longer version, based on a relatively pure single cell population of monocytes, can be used. The isolation of the monocytes is carried out by magnetic separation, in which monoclonal mouse anti-human CD14 antibodies, specific to monocytes, are bound to magnetic beads.

First, 200 ml of human blood was drawn into syringes containing diluted heparin (final concentration = 2IU/cc). The syringes were placed on rockers to ensure thorough mixing. The blood was then aliquoted into Histopaque tubes to begin the cell separation. Each Histopaque tube held no more than 20 ml of whole blood. The tubes were then centrifuged (1875 rpm) at room temperature for 20 minutes. The Histopaque tubes very effectively separate whole blood into three distinguishable layers: red blood cells, mononuclear cells, and plasma. The top layer, which constitutes the plasma, was removed for either discarding or later use.

The mononuclear cell layer was removed with the aid of a pipet and transferred to 50 ml centrifuge tubes. The tubes were subsequently filled to 50 ml with buffer and centrifuged (1050 rpm) at room temperature for 10 minutes. The resulting supernatant was discarded and the cell pellet was resuspended in 15 ml of RPMI growth medium (Sigma R-7509). A coulter counter was used to determine the total white blood cell density (cells/µl) in the 15 ml cell solution.

If a monocyte cell population is desired, following the cell count determination, the tube containing the cells should be centrifuged at 1050 rpm for 10 minutes at room temperature, and the resulting supernatant discarded. The cell pellet is then resuspended in 80 µl magnetic isolation buffer per 10⁷ total cells. Twenty µl of MACS CD14 microbeads are then added per 10⁷ total cells, and the cell and bead mixture is mixed well and incubated for 15 minutes in the refrigerator at 6-12°C. The cells are then washed by adding magnetic isolation buffer (10-20x the original volume) and centrifuged at 1050 rpm for 10 minutes.

Next, the supernatant is carefully discarded and the pellet is resuspended in 500 µl of buffer for every 10⁸ cells. One of the following positive selection columns is chosen: MS+ for up to 10⁸ positive cells (2x10⁸ total cells); or LS+ for up to 10⁸ positive cells (2×10⁹ total cells). In setting up the MiniMACS, the columns are prepared by washing with the appropriate amounts of buffer: MS+ = 500µl, LS+ = 3.0 ml. The magnetic cell suspension is then applied to the column with appropriate amounts of buffer (MS+ for a 0.5-1.0 ml volume, LS+ for a 1-10 ml volume). After allowing the negative cells to pass, the column is rinsed three times with the appropriate amount of buffer: MS+ = 3 × 500 µl, LS+ = 3 × 3.0 ml. With each wash the entire amount of buffer is allowed to flow through before the next wash step is started. After removing the column from the magnet, a tube for the collection of the positive cells is placed under the column. Buffer (MS+ = 1 ml, LS+ =5 ml) is then used to flush out the positive cells with the aide of a plunger. Finally, a coulter counter is used to determine the monocyte density (cells/µl) after resuspending the flushed out cell solution in 15 ml of RPMI growth medium.

Cell seeding was performed by adding monocytes, at 500,000 to 1,000,000 cells per well, to the test wells. One of the following two testing schemes may be adopted: (1) cell preconditioning by adding test agents/drugs to the cells for 24 hours at 37°C, followed by a LPS (10 µg/mL per well) treatment for an additional 24 hours at 37°C; or (2) the cells, test agents/drugs, and LPS are combined simultaneously and incubated at 37°C for 24 hours.

To assay for cytokine concentration, the solution in each test well was transferred to microcentrifuge tubes and centrifuged (3000 rpm) for 5 minutes to pellet any cells that were drawn from the wells. The supernatants were then transferred to fresh tubes and stored in a minus 85°C freezer until used. The cytokine content in the stored supernatants was determined using commercially available cytokine ELISA- (enzyme linked immunosorbent assays) based assays from, for example, R&D Systems, Minneapolis, MN, using the manufacturer's instructions. Essentially, the procedure was as follows: first, 200 µl of the test samples were added to each well of the microplates. The wells of the microplates were precoated with monoclonal antibodies specific to the cytokine being analyzed. All lipopolysaccharide-(LPS) treated samples were diluted 1:10 in RPMI growth media prior to the assay. The plates were covered and incubated for 2 hours at room temperature. Each well was then washed three times with wash buffer (350 µl/well), and 200 µl of cytokine conjugate were added to each well, covered, and incubated for 1 hour at room temperature. Next, 200 µl of substrate solution were added to each well and incubated for 20 minutes at room temperature. The color development in the wells was stopped by adding 50 µl of stop solution to each well. The optical density readings of the color development were determined in a microplate reader set at 450 nm wavelength.

### II. MMP Assay Procedures

MMP assay development involved two separate determinations. The first set of experiments established the working conditions for the assay, namely the optimal cell density and the choice and concentration of stimulant. MMP-9 is secreted by monocytes, macrophages, and neutrophils, amongst other cells; however, under non-stimulatory *in vitro* conditions the basal level secretion of MMP-9 by these cells is very low. Thus, for the second set of experiments (performed to determine the inhibitory effects of potential drugs or reagents), the levels of MMP secretion were raised to sufficiently high levels so that drug-mediated differences could be detected.

Human-derived white blood cells were isolated from human blood by a procedure such as that described above. The total cell population was adjusted and added to 24 well polystyrene tissue culture plates in the following cell densities: 5 million, 2 million, 1 million and 0.5 million per well. The stimulants used to stimulate MMP secretion were PMA (phorbol 12-myristate 13-acetate, Sigma # P8139) at a working concentration of 100 nM; PHA (phythohemagglutinin, Sigma # L9017) at a working concentration of 10 µg/ml; and PMA (100nM) + PHA (10 µg/ml). Controls were run where no stimulation was performed and MMP activity was at basal cellular level. The cells were allowed to incubate for 24 hours at 37°C. The secreted MMP was determined by ELISA using a Quantikine Human MMP-9 (total) Immunoassay (R&D Systems, # DMP900).

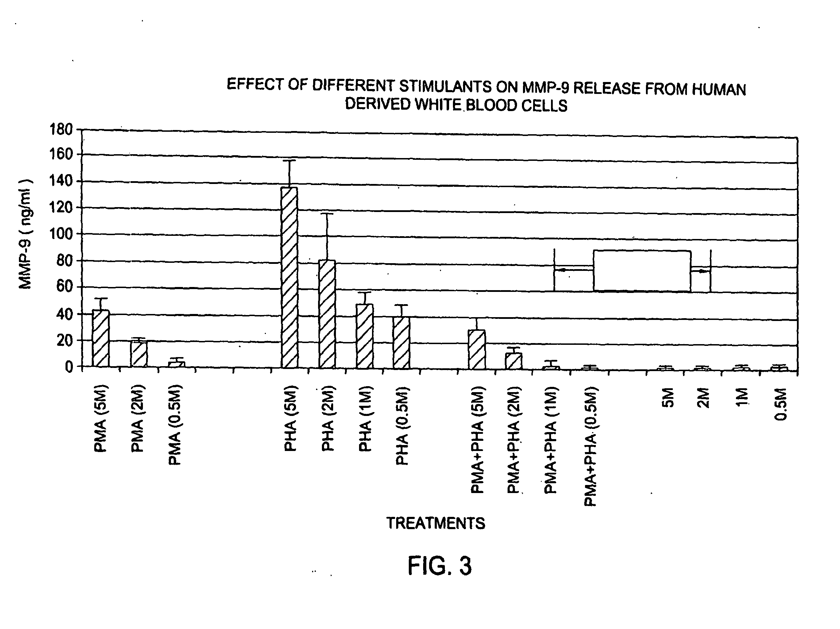

As expected, the basal expression of MMP-9 was observed to be very low (see Figure 3). Overall, enhanced expression levels were observed under all stimulated conditions (PMA or PHA), with increasing levels associated with increasing cell numbers. PHA alone was able to generate the most pronounced effect across all cell densities, with a greater than 200-fold increase in MMP-9 secretion over basal levels at the highest cell density of five million (5M) cells. Based on these results, 5 million cells and PHA were selected as appropriate assay conditions.

To perform an MMP assay to assess the effect of doxycycline on human-derived white blood cells, cells were seeded into 24 well tissue culture plates. The drug concentrations selected for the experiment were based on measured tissue levels of doxycycline following an oral dose of 100 mg. The cells were exposed to the drugs under two schemes. In scheme 1, the cells, the stimulant and the drug were all added at the same time and allowed to incubate for a period of 24 hours. In scheme 2 the cells were preconditioned with the drug for a period of 24 hours and then stimulated with PHA for an additional 24 hours. In this case the total experimental time duration was 48 hours. The seeding densities: 5 × 10⁶ cells per well and assay conditions were as follows: PHA at a working concentration of 10 µg/ml; doxycycline concentrations at 0, 2.5, 5, 10, 12, or 15 µM and either a 24 hour (scheme 1) or 48 hour (scheme 2) duration. MMP-9 analysis was performed using a Quantikine Human MMP-9 (total) Immunoassay (R&D Systems, # DMP900).

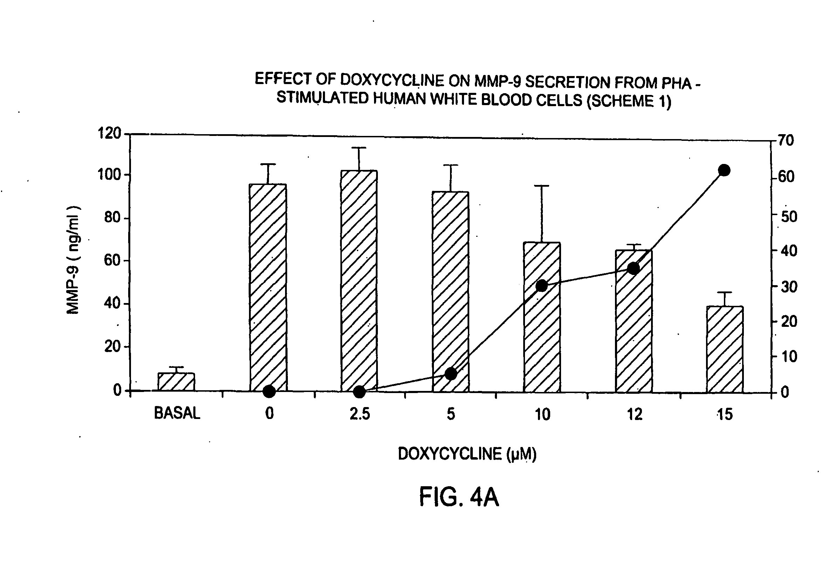

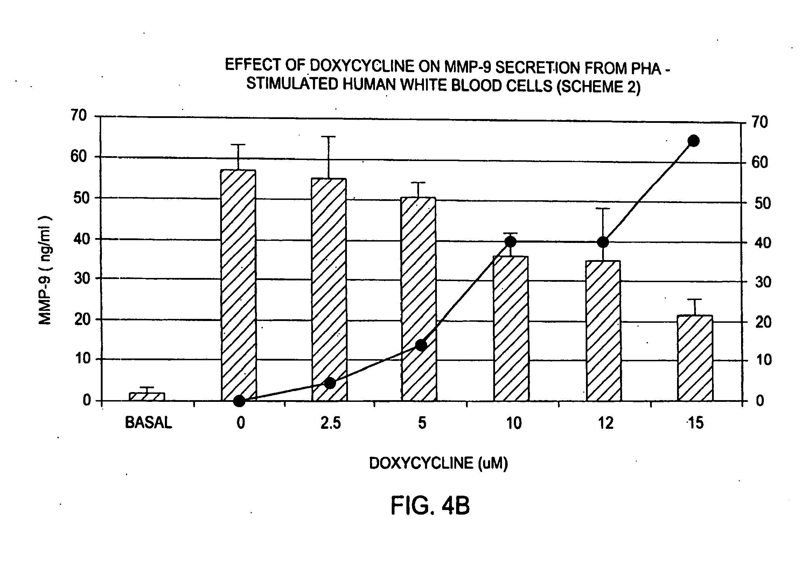

A dose-dependent inhibitory effect by doxycycline on MMP-9 expression was observed (see Figures 4A, 4B). The highest concentration of doxycycline (at 15 µM) registered an approximate inhibition of 60%. While the total amount of MMP-9 secretion was lower in scheme 2, there was no significant difference between the pattern and degree of inhibition between the two tested schemes.

### III. Combination Therapeutic Assay

A stability assay was first performed to ascertain whether doxycycline and dexamethasone react with one another. High concentrations of each solution were combined (DOX (56.9 ug/ml) + DEX (10 ug/ml)) and allowed to incubate for 4 days at 37°C in an orbital shaker. At the end of four days the concentrations of each drug in the mixture was determined by spectrophotometry and compared to the original concentrations. As seen in Table I, after 4 days in solution, there was only marginal deviation from the original concentrations with the DOX+DEX mixture. This suggests that the two drugs at the concentrations selected here were not reacting with each other and that the solution was fairly stable.

**Table 1**

| **Buffer** | **Drug** | **Initial time** | **Original Concentration** | **Later time** | **Final Concentration** |
|---|---|---|---|---|---|
| MES | DOX | 0 | 56.9 ug/ml | 4 days | ~ 56.7 ug/ml |
| | DEX | 0 | 10 ug/ml | 4 days | ~ 8.2 ug/ml |
| PBS | DOX | 0 | 56.9 ug/ml | 4 days | ~ 57.5/ml |
| | DEX | 0 | 10 ug/ml | 4 days | ~ 8.2 ug/ml |

| | | | | | |
|---|---|---|---|---|---|
| *UV-Vis readings of the combinations were complicated by the fact that peaks were observed at similar wavelengths for both drugs. For example: DOX, which has a maximum at 352nm, also registers a peak at 240nm. DEX also is read at 240nm. To separate the peaks calculations were made based on the following equation: A_{1M}=A₁ₓ+A_{iy} = E₁ₓbC_{x +} E_{1y}bC_{y} (where A=absorbance, m=mixture, x=DEX, y=DOX, b=path length (~1cm), E₁=extinction coefficient, C=concentration). There were 2 wavelengths (DEX at 240, DOX at 352), 2 unknowns and 2 equations.* | | | | | |

Next, to test the effect of therapeutics in combination, assays were performed to determine the effect of doxycycline alone (DOX at 5.69 ug/ml); dexamethasone alone (DEX at 1 ug/ml) and the effect of a combination of doxycycline and dexamethasone (DOX at 5.69 ug/ml) + DEX at 1 ug/ml). Dexamethasone is a potent agent with broad anti-inflammatory (against cytokines, chemokines, prostaglandins, etc.) and immunosuppressive properties. Dexamethasone also has known anti-angiogenic properties. Doxycycline was used as the MMP inhibitor for these studies.

Briefly, human-derived white blood cells were placed in a 24 well plate and exposed to a stimulant (PHA or lipopolysaccharide (LPS)) and the three different treatment regimes for a period of 24 hours at 37°C. The supernatants were then collected and assayed for MMP-9 and IL-1 content.

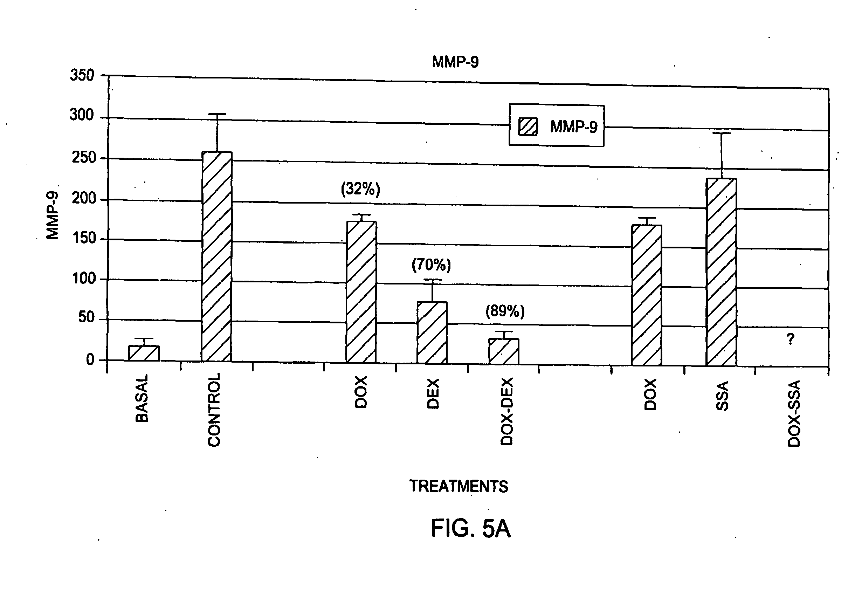

The results of the MMP assay are shown in Figure 5A. DOX, compared to the control, inhibited MMP-9 by approximately 32%, a level that has been observed consistently. DEX inhibited MMP-9 to a greater degree. The mixture of DOX+DEX generated the greatest effect, inhibiting MMP-9 expression by almost 90%, demonstrating that the mixture of the two drugs generates a synergistic effect that is greater than the action of each drug alone.

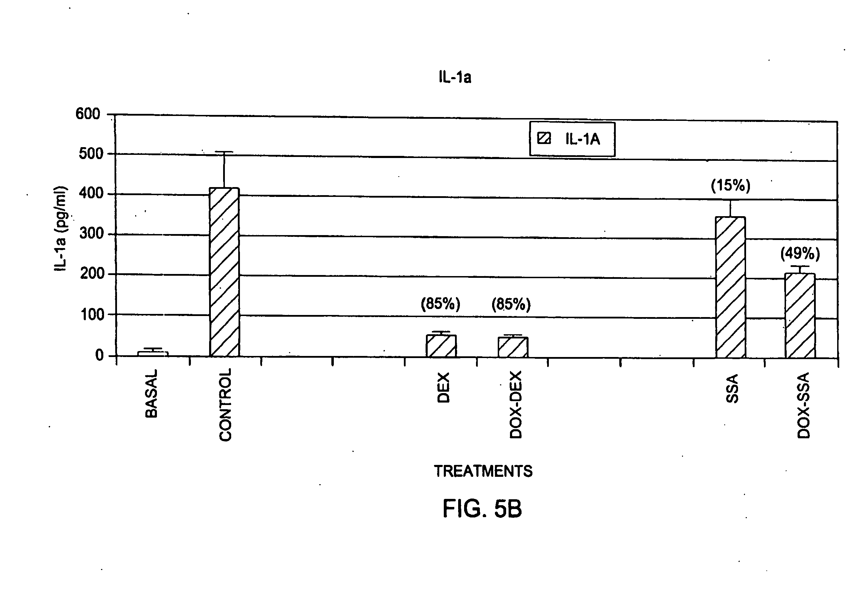

The results of the IL-α assay are shown in Figure 5B. As expected, DEX inhibited IL expression by approximately 85%, when compared to the control. The combination of DOX+DEX did not further inhibit the expression, suggesting that while DOX had no effect on inflammatory cytokine expression, it does not interfere with DEX's ability to function.

While the present invention has been described with reference to specific embodiments, it should be understood by those skilled in the art that various changes may be made and equivalents may be substituted within the scope of the claims.

## Claims

1. An intravascular treatment device, comprising:
a stent graft locatable adjacent to an aneurysmal site; wherein the stent graft includes a time release coating consisting essentially of a polymer or blend of polymers, an anti-inflammatory therapeutic agent and an matrix metalloproteinase inhibitor.

2. The treatment device of claim 1, wherein the polymer is biodegradable.

3. The treatment device of claim 2, wherein the polymer is collagen, gelatin, hyaluronic acid, starch, cellulose, cellulose derivatives, casein, dextran, polysaccharide, fibrinogen, poly(D,L-lactide), poly(D,L-lactide-co-glycolide), poly(glycolide), poly(hydroxybutyrate), poly(alkylcarbonate), poly(orthoesters), polyester, poly(hydroxyvaleric acid), polydioxanone, poly(ethylene terephthalate), poly(malic acid), poly(tartronic acid), polyanhydride, polyphosphazene, poly(amino acids), copolymers or combinations thereof.

4. The treatment device of claim 1, wherein the polymer is not biodegradable.

5. The treatment device of claim 4, wherein the polymer is poly(ethylene-vinyl acetate), silicone rubber, acrylic polymer, polyethylene, polypropylene, polyamide, nylon 6,6, polyurethane, poly(ester urethane), poly(ether urethanes, poly(ester-urea), polyethers (poly(ethylene oxide), poly(propylene oxide), pluronics, poly(tetramethylene glycol)), silicone rubber, or vinyl polymer, or copolymers or combinations thereof.

6. The treatment device of claim 1, wherein the polymer is poly(ethylene-vinyl acetate), polyurethane, poly (D,L-lactic acid) oligomers or polymers, poly (L-lactic acid) oligomers or polymers, poly (glycolic acid), copolymers of lactic acid and glycolic acid, poly (caprolactone), poly (valerolactone), polyanhydride, copolymers of poly (caprolactone) or poly (lactic acid) with a polyethylene glycol, or blends, admixtures, or copolymers or combinations thereof.

7. The treatment device of claim 1, wherein the polymer is hyaluronic acid, chitosan or fucans.

8. The treatment device of claim 1, wherein the polymer is a pH-sensitive polymer.

9. The treatment device of claim 8, wherein the pH-sensitive polymer is poly(acrylic acid) or its derivatives; poly(acrylic acid); poly(methyl acrylic acid), copolymers of poly(acrylic acid) and acrylmonomers; cellulose acetate phthalate; hydroxypropylmethylcellulose phthalate; hydroxypropyl methylcellulose acetate succinate; cellulose acetate trimellilate; chitosan, or copolymers or combinations thereof.

10. The treatment device of claim 1, wherein the polymer is a temperature-sensitive polymer.

11. The treatment device of claim 10, wherein the temperature-sensitive polymer is poly(N-methyl-N-n-propylacrylamide; poly(N-n-propylacrylamide); poly(N-methyl-N-isopropylacrylamide); poly(N-n-propylmethacrylamide; poly(N-isopropylacrylamide); poly(N,n-diethylacrylamide); poly(N-isopropylmethacrylamide); poly(N-cyclopropylacrylamide); poly(N-ethylmethyacrylamide); poly(N-methyl-N-ethylacrylamide); poly(N-cyclopropylmethacrylamide); poly(N-ethylacrylamide); hydroxypropyl cellulose; methyl cellulose; hydroxypropylmethyl cellulose; and ethylhydroxyethyl cellulose, or pluronics F-127; L-122; L-92; L-81; or L-61, or copolymers or combinations thereof.

12. The treatment device of claim 1, wherein the matrix metalloproteinase inhibitor is doxycycline, aureomycin, chloromycin, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, 6α-deoxy-5-hydroxy-4-dedimethylaminotetracycline, tetracyclinonitrile, 6-α-benzylthiomethylenetetracycline, 6-fluoro-6-demethyltetracycline, or 11-α-chlorotetracycline.

13. The treatment device of claim 12, wherein the matrix metalloproteinase inhibitor is doxycycline.

14. The treatment device of claim 1, wherein the anti-inflammatory therapeutic agent comprises a steroidal anti-inflammatory agent.

15. The treatment device of claim 14, wherein the steroidal anti-inflammatory agent is dexamethasone.

16. The treatment device of claim 1, wherein the anti-inflammatory therapeutic agent comprises a non-steroidal anti-inflammatory agent

17. The treatment device of any of the preceding claims, wherein the coating further comprises a cyclooxygenase-2 inhibitor.

18. The treatment device of claim 17, wherein the cyclooxygenase-2 inhibitor is Celecoxib, Rofecoxib, Parecoxib, green tea, ginger, tumeric, chamomile, Chinese gold-thread, barberry, baikal skullcap, Japanese knotweed, rosemary, hops, feverfew, oregano, piroxican, mefenamic acid, meloxican, nimesulide, diclofenac, MF-tricyclide, raldecoxide, nambumetone, naproxen, herbimycin-A, or etoicoxib.

19. The treatment device of any of the preceding claims, wherein the coating further comprises an anti-adhesion molecule.

20. The treatment device of any of the preceding claims, wherein the coating further comprises a beta blocker.

21. The treatment device of any of the preceding claims, wherein the coating further comprises an angiotensin converting enzyme.

22. The treatment device of any of the preceding claims, wherein the anti-inflammatory therapeutic agent and matrix metalloproteinase inhibitor is linked by an occlusion in the coating polymer.

23. The treatment device of any of claims 1 to 21, wherein the anti-inflammatory therapeutic agent and matrix metalloproteinase inhibitor is bound by covalent linkages to the polymer.

24. The treatment device of any of claims 1 to 21, wherein the anti-inflammatory therapeutic agent and matrix metalloproteinase inhibitor is contained in a microsphere associated with the polymer.

25. The treatment device of claim 22, wherein in microsphere is about 50 nm to 500 µm in size.

26. The treatment device of any of the preceding claims, wherein the coating is applied as a paste, thread, film or spray.

27. The treatment device of any of claims 24 to 26, wherein a spray is prepared from microspheres of about 0.1 µm to about 100 µm in size.

28. The treatment device of claim 26, wherein the film is from 10 µm to 5 mm thick.

29. The treatment device of any of the preceding claims, further comprising a second coating deposed over the time release coating.

30. The treatment device of claim 29, wherein there are at least two time release coatings, wherein each time release coating is separated by the second coating.

31. The treatment device of any of the preceding claims, wherein the time release coating releases from about 1% to about 25% of the therapeutic agent in the first 10 days.

## Patentansprüche

1. Vorrichtung zur intravaskulären Behandlung, umfassend:
einen Stent-Graft, der benachbart zu einer aneurismatischen Stelle angeordnet werden kann, wobei der Stent-Graft eine Retard-Beschichtung beinhaltet, die im Wesentlichen aus einem Polymer oder einer Mischung von Polymeren, einem entzündungshemmenden therapeutischen Wirkstoff und einem Hemmstoff der Matrixmetalloproteinase besteht.

2. Vorrichtung zur Behandlung nach Anspruch 1, wobei das Polymer biologisch abbaubar ist.

3. Vorrichtung zur Behandlung nach Anspruch 2, wobei das Polymer Kollagen, Gelatine, Hyaluronsäure, Stärke, Cellulose, Cellulose-Derivative, Casein, Dextran, Polysaccharide, Fibrinogen, Poly(D,L-Lactid), Poly (D,L-Lactid-co-glycolid), Poly(glycolid), Poly(hydroxybutyrat), Poly(alkylcarbonat), Poly(orthoester), Polyester, Poly(hydroxyvaleriansäure), Polydioxanon, Poly(ethylen-terephthalat), Poly(oxybernsteinsäure), Poly(hydroxymalonsäure), Polyanhydrid, Polyphosphazen, Poly(aminosäuren), Copolymere oder Kombinationen daraus umfasst.

4. Vorrichtung zur Behandlung nach Anspruch 1, wobei das Polymer nicht biologisch abbaubar ist.

5. Vorrichtung zur Behandlung nach Anspruch 4, wobei das Polymer Poly(ethylen-vinylacetat), Silikonkautschuk, Acrylpolymer, Polyethylen, Polypropylen, Polyamid, Nylon 6,6, Polyurethan, Poly(esterurethan), Poly(etherurethane), Poly(ester-harnstoff), Polyether (Poly (ethylenoxid), Poly(propylenoxid), Pluronics, Poly-(tetramethylenglycol)), Silikonkautschuk oder Vinylpolymer oder Copolymere oder Kombinationen daraus umfasst.

6. Vorrichtung zur Behandlung nach Anspruch 1, wobei das Polymer Poly(ethylen-vinylacetat), Polyurethan, Poly-(D,L-Milchsäure)-Oligomere oder -Polymere, Poly(L-Milchsäure)-Oligomere oder -Polymere, Poly(glycolsäure), Copolymere aus Milchsäure und Glycolsäure, Poly(caprolacton), Poly(valerolacton), Polyanhydrid, Copolymere aus Poly(caprolacton) oder Poly(milchsäure) mit einem Polyethylenglycol, oder Mischungen, Beimischungen oder Copolymere oder Kombinationen daraus umfasst.

7. Vorrichtung zur Behandlung nach Anspruch 1, wobei das Polymer Hyaluronsäure, Chitosan oder Fucane ist.

8. Vorrichtung zur Behandlung nach Anspruch 1, wobei das Polymer ein pH-sensitives Polymer ist.

9. Vorrichtung zur Behandlung nach Anspruch 8, wobei das pH-sensitive Polymer Poly(acrylsäure) oder seine Derivative; Poly(acrylsäure); Poly(methyl-acrylsäure), Copolymere aus Poly(acrylsäure) und Acrylmonomeren; Celluloseacetat-phthalat; Hydroxypropylmethylcellulosephthalat; Hydroxypropylmethylcelluloseacetatsuccinat; Celluloseacetat-trimellilat; Chitosan oder Copolymere oder Kombinationen daraus umfasst.

10. Vorrichtung zur Behandlung nach Anspruch 1, wobei das Polymer ein temperatursensitives Polymer ist.

11. Vorrichtung zur Behandlung nach Anspruch 10, wobei das temperatursensitive Polymer Poly(N-methyl-N-n-propyl-acrylamid; Poly(N-n-propylacrylamid); Poly(N-methyl-N-isopropylacrylamid); Poly(N-n-propylmethacrylamid; Poly(N-isopropylacrylamid); Poly(N,n-diethylacryl-amid); Poly(N-isopropylmethacrylamid); Poly(N-cyclopropylacrylamid); Poly(N-ethylmethyacrylamid); Poly(N-methyl-N-ethylacrylamid); Poly(N-cyclopropylmethacrylamid); Poly(N-ethylacrylamid); Hydroxypropylcellulose; Methylcellulose; Hydroxypropylmethylcellulose; und Ethylhydroxyethylcellulose oder Pluronics F-127; L-122; L-92; L-81; oder L-61, oder Copolymere oder Kombinationen daraus umfasst.

12. Vorrichtung zur Behandlung nach Anspruch 1, wobei der Matrixmetalloproteinase-Hemmstoff Doxycyclin, Aureomycin, Chloromycin, 4-Dedimethylaminotetracyclin, 4-Dedimethylamino-5-oxytetracyclin, 4-Dedimethyiamino-7-chlorotetracyclin, 4-Hydroxy-4-dedimethylaminotetracyclin, 5 a, 6-Anhydro-4-hydroxy-4-dedimethylaminotetracyclin, 6-Demethyl-6-deoxy-4-dedimethylaminotetracyclin, 4-Dedimethylamino-12a-deoxytetracyclin, 6α-Deoxy-5-hydroxy-4-dedimethylaminotetracyclin, Tetracyclinonitril, 6-α-Benzylthiomethylentetracyclin, 6-Fluoro-6-demethyltetracyclin oder 11-α-Chlorotetracyclin umfasst.

13. Vorrichtung zur Behandlung nach Anspruch 12, wobei der Matrixmetalloproteinase-Hemmstoff Doxycyclin ist.

14. Vorrichtung zur Behandlung nach Anspruch 1, wobei der therapeutische entzündungshemmende Wirkstoff einen steroidalen entzündungshemmenden Wirkstoff umfasst.

15. Vorrichtung zur Behandlung nach Anspruch 14, wobei der steroidale entzündungshemmende Wirkstoff Dexamethason ist.

16. Vorrichtung zur Behandlung nach Anspruch 1, wobei der therapeutische entzündungshemmende Wirkstoff einen nicht-steroidalen entzündungshemmenden Wirkstoff umfasst.

17. Vorrichtung zur Behandlung nach einem der vorangehenden Ansprüche, wobei die Beschichtung ferner einen Hemmstoff der Cyclooxygenase-2 umfasst.

18. Vorrichtung zur Behandlung nach Anspruch 17, wobei der Hemmstoff der Cyclooxygenase-2 Celecoxib, Rofecoxib, Parecoxib, Grüner Tee, Ingwer, Kurkuma, Kamille, Chinesischer Goldfaden, Berberitze, Baikal-Helmkraut, Japanischer Knöterich, Rosmarin, Hopfen, Mutterkraut, Oregano, Piroxican, Mefenaminsäure, Meloxican, Nimesulid, Diclofenac, MF-tricyclid, Raldecoxid, Nambumeton, Naproxen, Herbimycin-A oder Etoicoxib umfasst.

19. Vorrichtung zur Behandlung nach einem der vorangehenden Ansprüche, wobei die Beschichtung ferner ein Anti-Adhäsionsmolekül umfasst.

20. Vorrichtung zur Behandlung nach einem der vorangehenden Ansprüche, wobei die Beschichtung ferner einen Betablocker umfasst.

21. Vorrichtung zur Behandlung nach einem der vorangehenden Ansprüche, wobei die Beschichtung ferner ein Angiotensin-Converting-Enzym umfasst.

22. Vorrichtung zur Behandlung nach einem der vorangehenden Ansprüche, wobei der entzündungshemmende therapeutische Wirkstoff und der Matrixmetalloproteinase-Hemmstoff durch eine Okklusion in dem Beschichtungspolymer verbunden sind.

23. Vorrichtung zur Behandlung nach einem der Ansprüche 1 bis 21, wobei der entzündungshemmende therapeutische Wirkstoff und der Matrixmetalloproteinase-Hemmstoff über kovalente Bindungen mit dem Polymer verbunden sind.

24. Vorrichtung zur Behandlung nach einem der Ansprüche 1 bis 21, wobei der entzündungshemmende therapeutische Wirkstoff und der Matrixmetalloproteinase-Hemmstoff in einer Mikrokugel enthalten sind, die mit dem Polymer verbunden ist.

25. Vorrichtung zur Behandlung nach Anspruch 22, wobei eine Mikrokugel eine Größe von annähernd 50 nm bis 500 µm aufweist.

26. Vorrichtung zur Behandlung nach einem der vorangehenden Ansprüche, wobei die Beschichtung als eine Paste, ein Faden, ein Film oder ein Sprühmittel aufgetragen ist.

27. Vorrichtung zur Behandlung nach einem der Ansprüche 24 bis 26, wobei ein Sprühmittel aus Mikrokugeln mit einer Größe von annähernd 0,1 µm bis annähernd 100 µm hergestellt ist.

28. Vorrichtung zur Behandlung nach Anspruch 26, wobei der Film eine Dicke von 10 µm bis 5 mm aufweist.

29. Vorrichtung zur Behandlung nach einem der vorangehenden Ansprüche, die ferner eine zweite Beschichtung umfasst, welche über der Retard-Beschichtung aufgetragen ist.

30. Vorrichtung zur Behandlung nach Anspruch 29, wobei mindestens zwei Retard-Beschichtungen existieren, wobei jede Retard-Beschichtung durch die zweite Beschichtung getrennt ist.

31. Vorrichtung zur Behandlung nach einem der vorangehenden Ansprüche, wobei in den ersten 10 Tagen die Retard-Beschichtung von ungefähr 1 % bis ungefähr 25 % des therapeutischen Wirkstoffs freisetzt.

## Revendications

1. Dispositif de traitement intravasculaire, qui comprend :
une endoprothèse à positionner adjacente à un site d'anévrisme ; l'endoprothèse comprenant un revêtement retard essentiellement constitué par un polymère ou un mélange de polymères, un agent thérapeutique anti-inflammatoire et un inhibiteur des métalloprotéinases matricielles.

2. Dispositif de traitement selon la revendication 1, dans lequel le polymère est biodégradable.

3. Dispositif de traitement selon la revendication 2, dans lequel le polymère est le collagène, la gélatine, l'acide hyaluronique, l'amidon, la cellulose, les dérivés de la cellulose, la caséine, le dextrane, un polysaccharide, le fibrinogène, le poly(D,L-lactide), le poly(D,L-lactide-co-glycolide), le poly(glycolide), le poly (hydroxybutyrate), le poly(alkylcarbonate), les poly(orthoesters), le polyester, le poly(acide hydroxyvalérique), un polydioxanone, le poly(téréphtalate d'éthylène), le poly(acide malique), le poly(acide tartronique), un polyanhydride, un polyphosphazène, des poly(acides aminés), des copolymères ou des combinaisons de ceux-ci.

4. Dispositif de traitement selon la revendication 1, dans lequel le polymère n'est pas biodégradable.

5. Dispositif de traitement selon la revendication 4, dans lequel le polymère est le poly(éthylène-acétate de vinyle), le caoutchouc silicone, un polymère acrylique, le polyéthylène, le polypropylène, un polyamide, le nylon 6,6, le polyuréthane, le poly(ester uréthane), des poly(ester uréthanes), le poly(ester-urée), les polyéthers (poly(oxyde d'éthylène)), le poly(oxyde de propylène), pluronics, le poly(tétraméthylène glycol), le caoutchouc silicone, ou un polymère de vinyle, ou des copolymères ou des combinaisons de ceux-ci.

6. Dispositif de traitement selon la revendication 1, dans lequel le polymère est le poly(éthylène-acétate de vinyle), le polyuréthane, des oligomères ou polymères de poly(acide D,L-lactique), des oligomères ou polymères de poly(acide L-lactique), le poly(acide glycolique), des copolymères d'acide lactique et d'acide glycolique, le poly (caprolactone), le poly(valérolactone), un polyanhydride, des copolymères de poly(caprolactone) ou de poly(acide lactique) avec un polyéthylène glycol, ou des mélanges, ou des copolymères ou des combinaisons de ceux-ci.

7. Dispositif de traitement selon la revendication 1, dans lequel le polymère est l'acide hyaluronique, un chitosane ou des fucanes.

8. Dispositif de traitement selon la revendication 1, dans lequel le polymère est un polymère sensible au pH.

9. Dispositif de traitement selon la revendication 8, dans lequel le polymère sensible au pH est le poly(acide acrylique) ou ses dérivés ; le poly(acide acrylique) ; le poly(acide méthyl acrylique) ; des copolymères de poly(acide acrylique) et des monomères d'acryle ; l'acétate phtalate de cellulose ; le phtalate d'hydroxypropylméthylcellulose ; l'acétate succinate d'hydroxypropylméthylcellulose ; l'acétate trimellitate de cellulose ; le chitosane, ou des copolymères ou des combinaisons de ceux-ci.

10. Dispositif de traitement selon la revendication 1, dans lequel le polymère est un polymère sensible à la température.

11. Dispositif de traitement selon la revendication 10, dans lequel le polymère sensible à la température est le poly(N-méthyl-N-n-propylacrylamide) ; le poly(N-n-propylacrylamide) ; le poly(N-méthyl-N-isopropylacrylamide) ; le poly(N-n-propylméthacrylamide) ; le poly(N-isopropylacrylamide) ; le poly(N,n-diéthylacrylamide) ; le poly(N-isopropylméthacrylamide) ; le poly(N-cyclopropylacrylamide) ; le poly(N-éthylméthylacrylamide) ; le poly(N-méthyl-N-éthylacrylamide) ; le poly(N-cyclopropylméthacrylamide) ; le poly(N-éthylacrylamide) ; l'hydroxypropylcellulose ; la méthylcellulose ; l'hydroxypropylméthylcellulose ; et l'éthylhydroxyéthylcellulose, ou pluronics F-127 ; L-122 ; L-92 ; L-81 ; ou L-61, ou des copolymères ou des combinaisons de ceux-ci.

12. Dispositif de traitement selon la revendication 1, dans lequel l'inhibiteur des métalloprotéinases matricielles est la doxycycline, l'auréomycine, la chloromycine, la 4-dédiméthylaminotétracycline, la 4-dédiméthylamino-5-oxytétracycline, la 4-dédiméthylamino-7-chlorotétracycline, la 4-hydroxy-4-dédiméthylaminotétracycline, la 5 a, 6-anhydro-4-hydroxy-4-dédiméthylaminotétracycline, la 6-déméthyl-6-désoxy-4-dédiméthylaminotétracycline, la 4-dédiméthylamino-12a-désoxytétracycline, la 6α-désoxy-5-hydroxy-4-dédiméthylaminotétracycline, le tétracyclinonitrile, la 6-α-benzylthiométhylènetétracycline, la 6-fluoro-6-déméthyltétracycline, ou la 11-α-chlorotétracycline.

13. Dispositif de traitement selon la revendication 12, dans lequel l'inhibiteur des métalloprotéinases matricielles est la doxycycline.

14. Dispositif de traitement selon la revendication 1, dans lequel l'agent thérapeutique anti-inflammatoire comprend un agent anti-inflammatoire stéroïdien.

15. Dispositif de traitement selon la revendication 14, dans lequel l'agent anti-inflammatoire stéroïdien est la dexaméthasone.

16. Dispositif de traitement selon la revendication 1, dans lequel l'agent thérapeutique anti-inflammatoire comprend un agent anti-inflammatoire non stéroïdien.

17. Dispositif de traitement selon l'une quelconque des revendications précédentes, dans lequel le revêtement comprend en outre un inhibiteur de la cyclooxygénase 2.

18. Dispositif de traitement selon la revendication 17, dans lequel l'inhibiteur de la cyclooxygénase 2 est le célécoxib, le rofécoxib, le parécoxib, le thé vert, le gingembre, le curcuma, la camomille, le coptide chinois, le nerprun de Pureh, la scutellaire du Baïkal, la renouée japonaise, le romarin, le houblon, la grande camomille, l'origan, le piroxicane, l'acide méfénamique, le méloxicane, le nimésulide, le diclofénac, le MF-tricyclide, le raldecoxide, la nambumétone, le naproxène, l'herbimycine-A, ou l'étoricoxib.

19. Dispositif de traitement selon l'une quelconque des revendications précédentes, dans lequel le revêtement comprend en outre une molécule anti-adhésion.

20. Dispositif de traitement selon l'une quelconque des revendications précédentes, dans lequel le revêtement comprend en outre un bêta bloquant.

21. Dispositif de traitement selon l'une quelconque des revendications précédentes, dans lequel le revêtement comprend en outre une enzyme de conversion de l'angiotensine.

22. Dispositif de traitement selon l'une quelconque des revendications précédentes, dans lequel l'agent thérapeutique anti-inflammatoire et l'inhibiteur des métalloprotéinases matricielles sont liés par une occlusion dans le polymère de revêtement.

23. Dispositif de traitement selon l'une quelconque des revendications 1 à 21, dans lequel l'agent thérapeutique anti-inflammatoire et l'inhibiteur des métalloprotéinases matricielles sont liés par liaisons covalentes au polymère.

24. Dispositif de traitement selon l'une quelconque des revendications 1 à 21, dans lequel l'agent thérapeutique anti-inflammatoire et l'inhibiteur des métalloprotéinases matricielles sont contenus dans une microsphère associée au polymère.

25. Dispositif de traitement selon la revendication 22, dans lequel la microsphère est d'une taille d'environ 50 nm à 500 µm.

26. Dispositif de traitement selon l'une quelconque des revendications précédentes, dans lequel le revêtement est appliqué sous la forme d'une pâte, d'un fil, d'un film ou d'un spray.

27. Dispositif de traitement selon l'une quelconque des revendications 24 à 26, dans lequel un spray est préparé à partir de microsphères d'une taille d'environ 0,1 µm à environ 100 µm.

28. Dispositif de traitement selon la revendication 26, dans lequel le film est d'une épaisseur de 10 µm à 5 mm.

29. Dispositif de traitement selon l'une quelconque des revendications précédentes, comprenant en outre un deuxième revêtement déposé sur le revêtement retard.

30. Dispositif de traitement selon la revendication 29, dans lequel il y a au moins deux revêtements retards, chaque revêtement retard étant séparé par le deuxième revêtement.

31. Dispositif de traitement selon l'une quelconque des revendications précédentes, dans lequel le revêtement retard libère d'environ 1 % à environ 25 % de l'agent thérapeutique au cours des 10 premiers jours.
